(19) Europäisches Patentamt
European Patent Office
Office européen des brevets

(11) Veröffentlichungsnummer: **0 074 538**
**A1**

(12) # EUROPÄISCHE PATENTANMELDUNG

(21) Anmeldenummer: 82107874.8

(22) Anmeldetag: 27.08.82

(51) Int. Cl.³: C 07 D 253/06
A 01 N 43/64
//C07C121/34, C07C103/153,
C07C59/125

(30) Priorität: 07.09.81 DE 3135392

(43) Veröffentlichungstag der Anmeldung:
23.03.83 Patentblatt 83/12

(84) Benannte Vertragsstaaten:
AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Kranz, Eckart, Dr.
Am Acker 9
D-5600 Wuppertal 1(DE)

(72) Erfinder: Findeisen, Kurt, Dr.
In der Follmühle 10
D-5068 Odenthal 2(DE)

(72) Erfinder: Eue, Ludwig, Dr.
Paul-Klee-Strasse 36
D-5090 Leverkusen(DE)

(72) Erfinder: Schmidt, Robert R., Dr.
Im Waldwinkel 110
D-5060 Bergisch-Gladbach 2(DE)

(54) **Substituierte 6-Alkoxy-tert.-butyl-1,2,4-triazin-5-one, Verfahren zu ihrer Herstellung sowie ihre Verwendung als Herbizide.**

(57) Neue substituierte 6-Alkoxy-tert.-butyl-1,2,4-triazin-5-one der allgemeinen Formel

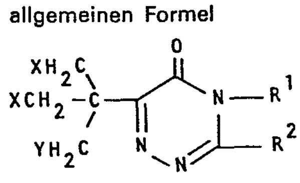

in welcher
$R^1$ für Amino oder die Gruppierung

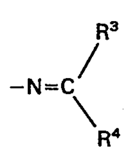 steht,

$R^2$ für Alkylthio, Alkylamino oder Dialkylamino steht,
$R^3$ für Wasserstoff oder Alkyl steht,
$R^4$ für Alkyl, Cycloalkyl, Cycloalkenyl oder gegebenenfalls substituiertes Phenyl steht,
X für Alkoxy steht sowie
Y und Z für Wasserstoff oder Alkoxy stehen,
ein Verfahren zu ihrer Herstellung und ihre Verwendung als Herbizide.

EP 0 074 538 A1

Croydon Printing Company Ltd.

0074538

BAYER AKTIENGESELLSCHAFT
5090 Leverkusen, Bayerwerk
Zentralbereich
Patente, Marken und Lizenzen

04.09.81
Bi/Fr
Ia

# Substituierte 6-Alkoxy-tert.-butyl-1,2,4-triazin-5-one, Verfahren zu ihrer Herstellung sowie ihre Verwendung als Herbizide

Die vorliegende Erfindung betrifft neue 6-Alkoxy-tert.-butyl-1,2,4-triazin-5-one, ein Verfahren zu ihrer Herstellung sowie ihre Verwendung als Herbizide, insbesondere als selektive Herbizide.

Es ist bereits bekannt geworden, daß 4-Amino-6-tert.-butyl-1,2,4-triazin-5-one, wie z.B. 3-Ethyl-4-cyclohexylidenamino-6-tert.-butyl-1,2,4-triazin-5-on, als Herbizide verwendet werden können (vgl. DE-OS 22 38 206 [LeA 14 483]). In bestimmten Kulturen ist jedoch ein selektiver Einsatz der vorbekannten Triazinone nicht möglich, da es infolge der durchweg hohen herbiziden Potenz dieser Stoffgruppe auch bei bestimmten Nutzpflanzen zu Schäden kommen kann; die Verträglichkeit gegenüber den vorbekannten Triazinonen ist demnach bei verschiedenen Nutzpflanzen nicht ausreichend.

Es wurden neue substituierte 6-Alkoxy-tert.-butyl-1,2,4-triazin-5-one der allgemeinen Formel I

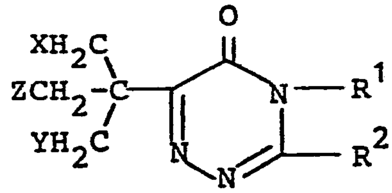

(I)

in welcher

$R^1$ für Amino oder die Gruppierung 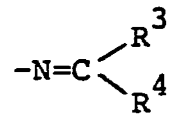 steht,

$R^2$ für Alkylthio, Alkylamino oder Dialkylamino steht,

$R^3$ für Wasserstoff oder Alkyl steht,

$R^4$ für Alkyl, Cycloalkyl, Cycloalkenyl oder gegebenenfalls substituiertes Phenyl steht,

X für Alkoxy steht sowie

Y und Z für Wasserstoff oder Alkoxy stehen,

aufgefunden.

Weiterhin wurde gefunden, daß man die substituierten 6-Alkoxy-tert.-butyl-1,2,4-triazin-5-one der Formel (I) erhält, wenn man in einer <u>ersten Stufe</u> Alkoxypivaloylcyanide der Formel II

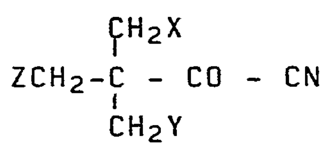

(II)

in welcher

X,Y und Z die oben angegebene Bedeutung haben,

(a) gegebenenfalls in Gegenwart einer flüssigen Carbonsäure als Lösungsmittel, mit einer anorganischen Säure umsetzt oder

(b) an ein Carboniumion addiert, das aus einem Olefin, wie z.B. Isobutylen, oder einem tertiären Alkohol, wie z.B. tert.-Butanol, mit einer starken Säure, wie insbesondere Schwefelsäure, gebildet wird, und anschließend der Hydrolyse unterwirft (sog. RITTER-REAKTION)

und

die hierbei entstehenden substituierten Trimethylbrenztraubensäureamide der Formel IIIa

$$ZCH_2 - \underset{CH_2Y}{\overset{CH_2X}{\underset{|}{\overset{|}{C}}}} - CO\text{-}CO - NHR \qquad \text{(IIIa)}$$

in welcher

X,Y und Z die oben angegebene Bedeutung haben und

R für Wasserstoff (erhältlich nach Variante a) oder Alkyl, insbesondere tert.-Butyl (erhältlich nach Variante b), steht,

in einer <u>zweiten Stufe</u> in an sich bekannter Weise entweder direkt in der anfallenden Lösung oder nach Zwischenisolierung, gegebenenfalls nach vorheriger Verseifung zu den freien substituierten Trimethylbrenztraubensäuren der Formel IIIb

$$ZCH_2 - \underset{CH_2Y}{\overset{CH_2X}{\underset{|}{\overset{|}{C}}}} - CO - COOH \qquad \text{(IIIb)}$$

in welcher

X,Y und Z die oben angegebene Bedeutung haben,

mit Thiocarbohydrazid der Formel IV

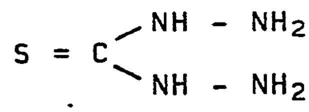

(IV)

in wässriger bzw. in wässrig-saurer Lösung, gegebenenfalls in Gegenwart eines organischen Verdünnungsmittels, zu 6-Alkoxy-tert.-butyl-3-mercapto-1,2,4-triazin-5-onen der Formel V

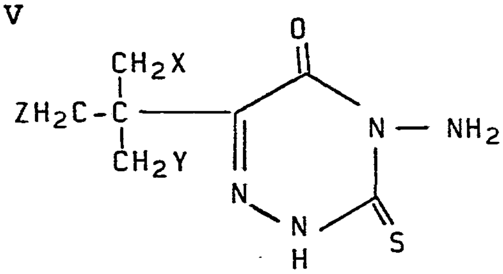

(V)

in welcher

X,Y und Z die oben angegebene Bedeutung haben,

umsetzt, diese in einer <u>dritten Stufe</u> in bekannter Weise in alkalischer Lösung mittels Alkylhalogenid, vorzugsweise Alkyljodid oder -bromid, zu 6-Alkoxy-tert.-butyl-3-alkylthio-1,2,4-triazin-5-onen der Formel Ia

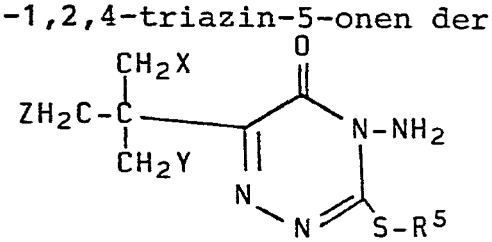

(Ia)

in welcher

X,Y und Z die oben angegebene Bedeutung haben und

$R^5$ für Alkyl, vorzúgsweise mit 1 bis 4 Kohlenstoffatomen steht,

alkyliert, diese gegebenenfalls in einer <u>vierten Stufe</u> mit Aminen der Formel VI

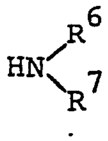

(VI)

in welcher

$R^6$ für Wasserstoff oder Alkyl, vorzugsweise mit 1 bis 6 Kohlenstoffatomen, steht und

$R^7$ für Alkyl, vorzugsweise mit 1 bis 6 Kohlenstoffatomen, steht,

in Gegenwart eines Verdünnungsmittels umsetzt und gegebenenfalls in einer <u>fünften Stufe</u> die gemäß der dritten bzw. vierten Stufe entstandenen 6-Alkoxy-tert.-butyl-4-amino-1,2,4-triazin-5-one der Formel Ib

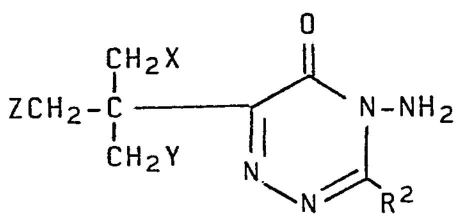

(Ib)

in welcher

$R^2$, X, Y und Z die oben angegebene Bedeutung haben,

mit Carbonylverbindungen der Formel VII

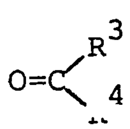

(VII)

in welcher

$R^3$ und $R^4$ die oben angegebene Bedeutung haben,

gegebenenfalls in Gegenwart eines Verdünnunsgmittels und gegebenenfalls in Gegenwart eines basischen oder sauren Katalysators umsetzt; die Carbonylverbindungen der Formel (VII) können dabei gegebenenfalls in Form von Acetalen bzw. Ketalen eingesetzt werden.

Außerdem wurde gefunden, daß die substituierten 6-Alkoxy-tert.-butyl-1,2,4-triazin-5-one der Formel (I) gute herbizide, insbesondere selektiv-herbizide Eigenschaften aufweisen.

Ueberraschenderweise zeigen die erfindungsgemäßen Verbindungen gegenüber dem bekannten 3-Ethyl-4-cyclohexylidenamino-6-tert.-butyl-1,2,4-triazin-5-on, welches chemisch und wirkungsmäßig eine naheliegende Verbindung ist, neben einer besseren allgemeinen herbiziden Wirkung, insbesondere eine bessere Verträglichkeit bei wichtigen Kulturpflanzen, wie bei Mais und Baumwolle. Die erfindungsgemäßen Wirkstoffe stellen somit eine Bereicherung der herbiziden Mittel, insbesondere der selektiven chemischen Unkrautbekämpfung dar.

Die erfindungsgemäßen substituierten 6-Alkoxy-tert.-butyl-1,2,4-triazin-5-one sind durch die Formel (I) allgemein definiert. In dieser Formel stehen vorzugsweise

$R^1$ für Amino ($NH_2$) oder die Gruppierung $-N{=}CR^3R^4$;

$R^2$ für geradkettiges oder verzweigtes Alkylthio mit 1 bis 4 Kohlenstoffatomen, sowie für Alkylamino und Dialkylamino mit jeweils 1 bis 6 Kohlenstoffatomen in jedem Alkylteil;

$R^3$ für Wasserstoff oder geradkettiges und verzweigtes Alkyl mit 1 bis 6 Kohlenstoffatomen;

$R^4$ für geradkettiges oder verzweigtes Alkyl mit 1 bis 6 Kohlenstoffatomen, für Cycloalkyl mit 3 bis 6 Kohlenstoffatomen oder Cycloalkenyl mit 5 oder 6 Kohlenstoffatomen, für gegebenenfalls substituiertes Phenyl, wobei als Substituenten beispielsweise genannt seien: Halogen, Alkyl mit 1 bis 4 Kohlenstoffatomen, Halogenalkyl mit 1 bis 2 Kohlenstoffatomen und bis zu 5 gleichen oder verschiedenen Halogenatomen (wie vorzugsweise Fluor- und Chloratomen), Alkoxy und Alkylthio mit jeweils 1 bis 2 Kohlenstoffatomen, Cyano und Nitro;

X für geradkettiges oder verzweigtes Alkoxy mit 1 bis 4 Kohlenstoffatomen;

Y und Z für Wasserstoff oder geradkettiges oder verzweigtes Alkoxy mit 1 bis 4 Kohlenstoffatomen.

Besonders bevorzugt sind diejenigen Verbindungen der Formel (I), in denen

$R^1$ die oben angegebene Bedeutung hat;

$R^2$ für Methyl-, Ethyl- oder Propylthio, ferner für Methyl-, Ethyl-, Propyl-, Hexylamino sowie Dimethyl-, Diethyl- und Ethylmethylamino steht;

$R^3$ für Wasserstoff oder geradkettiges und verzweigtes Alkyl mit 1 bis 4 Kohlenstoffatomen steht;

$R^4$ für geradkettiges oder verzweigtes Alkyl mit 1 bis 4 Kohlenstoffatomen, Cyclohexyl, Cyclohexenyl oder für gegebenenfalls substituiertes Phenyl steht, wobei als Substituenten genannt seien: Fluor, Chlor, Methyl, Ethyl und Nitro;

X für Methoxy, Ethoxy oder Isopropoxy steht;

Y und Z für Wasserstoff, Methoxy, Ethoxy oder Isopropoxy stehen.

Verwendet man beispielsweise Methoxypivaloylcyanid als Ausgangsstoff in der ersten Stufe, setzt in der zweiten Stufe die entsprechende freie Säure mit Thiocarbohydrazid um und verwendet in der dritten Stufe Methylbromid als Alkylierungsmittel, so kann der Reaktionsablauf durch das folgende Formelschema wiedergegeben werden:

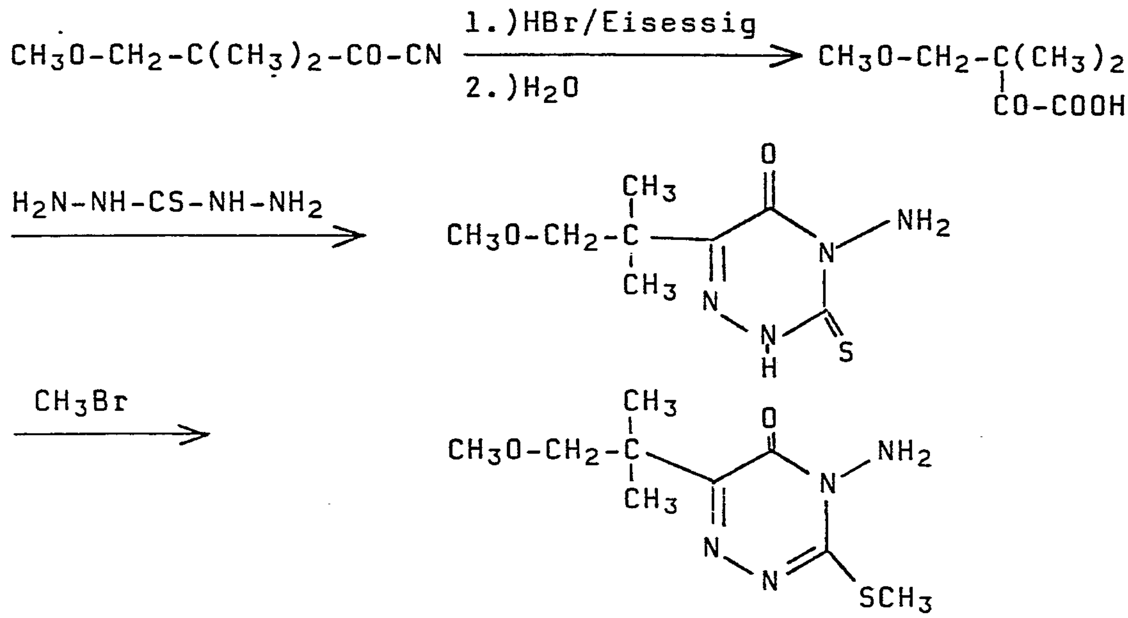


Verwendet man das so erhaltene 4-Amino-6-methoxy-tert.-butyl-3-methylthio-1,2,4-triazin-5-on und Dimethylamin als Ausgangsstoffe für die vierte Stufe, so kann der Reaktionsablauf durch das folgende Formelschema wiedergegeben werden:

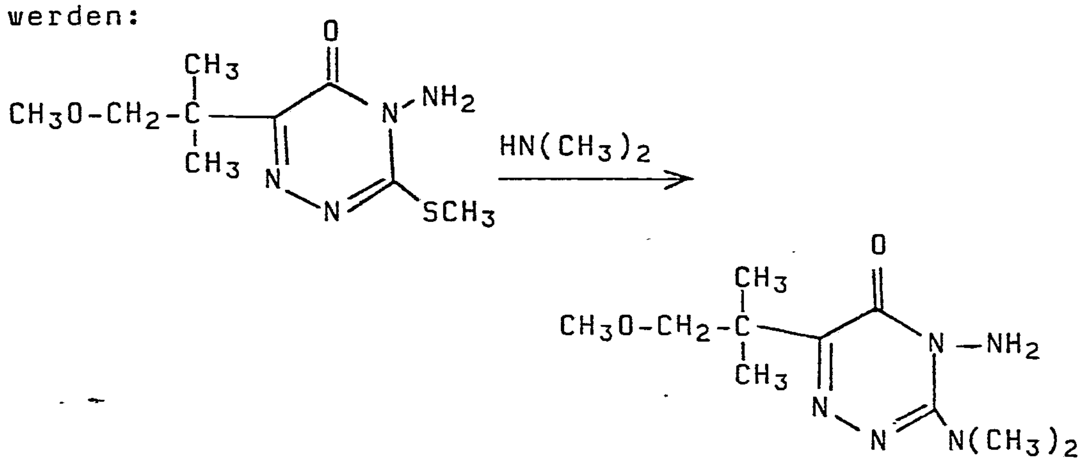

Verwendet man beispielsweise 4-Amino-6-methoxy-tert.-butyl-3-methylthio-1,2,4-triazin-5-on und Aceton als Ausgangsstoffe für die fünfte Stufe, so kann der Reaktionsablauf durch das folgende Formelschema wiedergegeben werden:

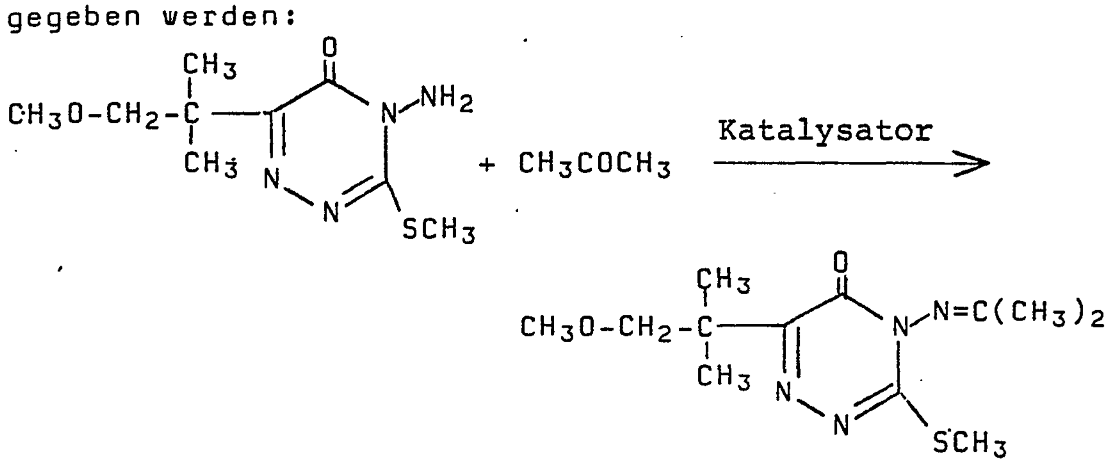


Die für das erfindungsgemäße Verfahren als Ausgangsstoffe zu verwendenden Alkoxy-pivaloylcyanide sind durch die Formel (II) allgemein definiert. In dieser Formel stehen X, Y und Z vorzugsweise für die Reste, die bereits im Zusammenhang mit der Beschreibung der erfindungsgemäßen Stoffe der Formel (I) vorzugsweise für diese Substituenten genannt wurden.

Die Alkoxypivaloylcyanide der Formel (II) sind noch nicht bekannt. Sie können erhalten werden, indem man die entsprechenden Alkoxypivaloylhalogenide bzw. -anhydride der Formeln VIIIa bzw. VIIIb

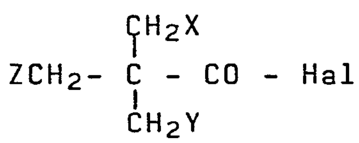 (VIIIa)

bzw.

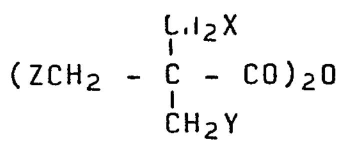 (VIIIb)

in welchen

Hal für Halogen, vorzugsweise Chlor oder Brom, steht und

X,Y und Z die oben angegebene Bedeutung haben,

mit Trimethylsilylcyanid gegebenenfalls in Gegenwart eines Verdünnungsmittels umsetzt. Trimethylsilylcyanid, $(CH_3)_3Si-CN$, ist bekannt (vgl. z.B. Synthesis 1979, Seiten 522 und 523).

Dieses Verfahren zur Herstellung der Alkoxypivaloylcyanide der Formel (II) wird gegebenenfalls in Gegenwart eines Verdünnungsmittels durchgeführt. Hierzu gehören vorzugsweise inerte organische Lösungsmittel, wie Ketone, z.B. Aceton und Methylethylketon; Nitrile, z.B. Acetonitril; Ether, z.B. Tetrahydrofuran oder Dioxan; sowie aromatische Kohlenwasserstoffe, z.B. Benzol, Toluol oder Xylol. - Vorzugsweise wird ohne Lösungsmittel gearbeitet.

Die Reaktionstemperaturen können bei der Durchführung dieses Verfahrens in einem größeren Bereich variiert werden. Im allgemeinen arbeitet man zwischen 50 und 250°C, vorzugsweise zwischen 80 und 180°C.

Bei der Durchführung dieses Verfahrens arbeitet man vorzugsweise mit äquivalenten Mengen an Ausgangsstoffen. Die Isolierung der Verbindungen der Formel (II) erfolgt in üblicher Weise.

Die Alkoxypivaloylhalogenide bzw. -anhydride der Formeln (VIIIa) bzw. (VIIIb) sind bekannt, bzw. können sie nach bekannten Verfahren hergestellt werden.

Ebenfalls allgemein bekannt sind die Amine der Formel (VI), die Carbonylverbindungen der Formel (VII), d.h. Aldehyde und Ketone, sowie das Thiocarbohydrazid der Formel (IV).

Die erste Stufe des erfindungsgemäßen Verfahrens kann in Abwesenheit oder in Gegenwart einer unter den Reaktionsbedingungen flüssigen, niederaliphatischen Carbonsäure als Lösungsmittel durchgeführt werden. Als solche Lösungsmittel kommen vorzugsweise Carbonsäuren mit 1 bis 6 Kohlenstoffatomen, wie Essigsäure, Propionsäure oder Ameisensäure, infrage.

Die erste Stufe des erfindungsgemäßen Verfahrens wird mit Hilfe einer starken anorganischen Säure durchgeführt. Hierzu gehören vorzugsweise die Halogenwasserstoffsäuren, wie Chlorwasserstoff und Bromwasserstoff, sowie konzentrierte Schwefelsäure.

Die Reaktionstemperaturen können bei der Durchführung dieser Verfahrensstufe in einem größeren Bereich variiert werden. Im allgemeinen arbeitet man zwischen -20 und +50°C, vorzugsweise zwischen 0 und +40°C.

Bei der Durchführung der ersten Stufe des erfindungsgemäßen Verfahrens setzt man auf 1 Mol Alkoxy-pivaloylcyanid der Formel (II) vorzugsweise 1 bis 10 Mol an anorganischer Säure ein.

Die zweite Stufe des erfindungsgemäßen Verfahrens erfolgt vorzugsweise ohne vorherige Zwischenisolierung der substituierter Trimethylbrenztraubensäureamide der Formel (IIIa) und nach Verseifung derselben in üblicher Weise zu freien Säuren der Formel (IIIb).

Die zweite Stufe des erfindungsgemäßen Verfahrens wird in wässriger Lösung bzw. in Gegenwart einer wässrig-sauren Lösung wie einer halogenwasserstoffsauren, vorzugsweise einer salzsauren, oder einer schwefelsauren Lösung durchgeführt.

Wird in Gegenwart eines organischen Lösungsmittels gearbeitet, so kommen alle üblichen organischen Lösungsmittel infrage, wie insbesondere Dimethylformamid.

Die Reaktionstemperaturen können bei der Durchführung der zweiten Stufe des erfindungsgemäßen Verfahrens in einem größeren Bereich variiert werden. Im allgemeinen arbeitet man zwischen 0 und 120°C, vorzugsweise zwischen 0 und 100°C.

Bei der Durchführung der zweiten Stufe des erfindungsgemäßen Verfahrens setzt man die Ausgangsstoffe vorzugsweise in molaren Mengen bzw. mit einem Ueberschuß an Thiocarbohydrazid der Formel (IV) ein. Die Isolierung der Zwischenprodukte der Formel (V) erfolgt in üblicher Weise.

Die dritte Stufe des erfindungsgemäßen Verfahrens wird in Gegenwart einer Base durchgeführt. Hierbei werden vorzugsweise Alkalihydroxide,wie Natriumhydroxid, in wäßriger Lösung oder Alkalialkoholate, wie Natriummethylat, wobei überschüssiger Alkohol als Lösungsmittel verwendet wird, eingesetzt.

Die Reaktionstemperaturen können bei der Durchführung der dritten Stufe des erfindungsgemäßen Verfahrens in einem größeren Bereich variiert werden. Im allgemeinen arbeitet man zwischen 0 und 100°C, vorzugsweise zwischen 0 und 50°C.
Bei der Durchführung der dritten Stufe des erfindungsgemäßen Verfahrens setzt man auf 1 Mol Zwischenprodukt der Formel (V) vorzugsweise 1 bis 1,5 Mol Alkylierungsmittel ein. Die Isolierung der Zwischenprodukte bzw. Endprodukte der Formel (Ia) erfolgt in üblicher Weise.

Als Verdünnungsmittel kommen für die vierte Stufe des erfindungsgemäßen Verfahrens vorzugsweise organische Lösungsmittel infrage, wie insbesondere Isopropanol/Eisessig.

Die Reaktionstemperaturen können bei der Durchführung der vierten Stufe des erfindungsgemäßen Verfahrens in einem größeren Bereich variiert werden. Im allgemeinen arbeitet man zwischen 20 und 180°C, vorzugsweise zwischen 40 und 150°C.

Bei der Durchführung der vierten Stufe des erfindungsgemäßen Verfahrens setzt man auf 1 Mol der Verbindung der Formel (Ia) vorzugsweise 1 bis 3 Mol Amin der Formel (VI) ein. Die Isolierung der Endprodukte erfolgt in üblicher Weise.

Als Verdünnungsmittel kommen für die fünfte Stufe des erfindungsgemäßen Verfahrens alle inerten organischen Lösungsmittel infrage. Hierzu gehören vorzugsweise Alkohle, wie Methanol, Ethanol und tert.-Butanol; Ether, wie insbesondere Tetrahydrofuran und Dioxan; halogenierte Kohlenwasserstoffe, wie Methylenchlorid; sowie aromatische Kohlenwasserstoffe, wie Benzol, Toluol oder Xylol.

Die fünfte Stufe des erfindungsgemäßen Verfahrens wird gegebenenfalls in Gegenwart eines basischen oder eines sauren Katalysators durchgeführt. Hierbei können alle üblichen basischen Katalysatoren eingesetzt werden, wie z.B. Dimethylbenzylamin. Als saure Katalysatoren kommen insbesondere p-Toluolsulfonsäure oder saure Kationenaustauscher in Betracht.

Die Reaktionstemperaturen können bei der Durchführung der fünften Stufe des erfindungsgemäßen Verfahrens in einem größeren Bereich variiert werden. Im allgemeinen arbeitet man bei Temperaturen zwischen -50 und +150°C, vorzugsweise zwischen -10 und +120°C.

Bei der Durchführung der fünften Stufe des erfindungsgemäßen Verfahrens setzt man vorzugsweise auf 1 Mol 6-Alkoxy-tert.-butyl-4-amino-1,2,4-triazin-5-on der Formel (Ib) 1 bis 2 Mol einer Carbonylverbindung der Formel (VII) und gegebenenfalls 0,01 bis 0,1 Mol Katalysator ein. Die Isolierung der Verbindungen der Formel (I) erfolgt in üblicher Weise.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Bleocharis, Scirpus, Paspalum, Ischeamum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Wein-, Citrus-, Nuss-, Bananen-, Kaffee-, Tee-, Gummi-, Oelpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen neben einer sehr guten allgemeinen herbiziden Wirkung eine gute Verträglichkeit gegenüber Nutzpflanzen. So ist es möglich, wichtige Schadgräser selektiv in wichtigen Kulturpflanzen, wie z.B. in Mais und Baumwolle zu bekämpfen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe, Feinstverkapselungen in polymeren Stoffen.
Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:
z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminium und Silicate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen

Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden oder Pflanzenwachstumsregulatoren zur Unkrautbekämpfung bzw. als Pflanzenwuchsregulatoren Verwendung finden, wobei Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Eumulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei einer Anwendung als Herbizide sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die angewandte Wirkstoffmenge kann in einem größeren Bereich schwanken. Sie hängt im wesentlichen ab von der Art des gewünschten Effektes. Im allgemeinen liegen die Aufwandmengen zwischen o,1 und 10 kg Wirkstoff pro Hektar Bodenfläche, vorzugsweise zwischen o,1 und 5 kg pro ha.

Die nachfolgenden Beispiele dienen zur weiteren Erläuterung der Erfindung.

Herstellungsbeispiele

Beispiel 1

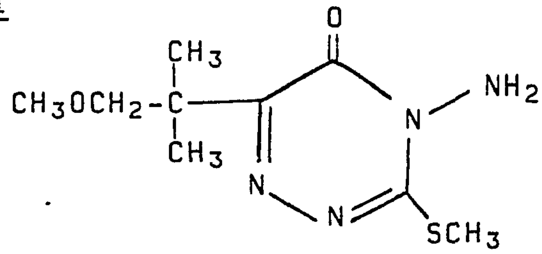

(1)

1. Stufe

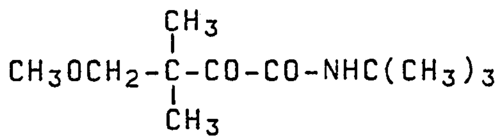

70,5g (0,5 Mol) Methoxypivaloylcyanid werden zu einer Lösung von 74g (1 Mol) tert.-Butanol in 35 ml Methylenchlorid gegeben. Dazu werden innerhalb von 10 Minuten bei 0 - 5°C 75g konzentrierte Schwefelsäure getropft. Man läßt die Innentemperatur auf Raumtemperatur ansteigen und rührt 4 Stunden nach. Anschließend wird die Reaktionslösung auf 200-300g Eis gegossen und zweimal mit Methylenchlorid extrahiert. Die vereinigten organischen Phasen werden mit Wasser gewaschen, über Natriumsulfat getrocknet und eingeengt. Man erhält 89,1 g (83 % der Theorie) Dimethylmethoxymethyl-brenztraubensäure-N-tert.-butyl-amid als hellgelbes Oel, das direkt weiter umgesetzt wird.

2. Stufe

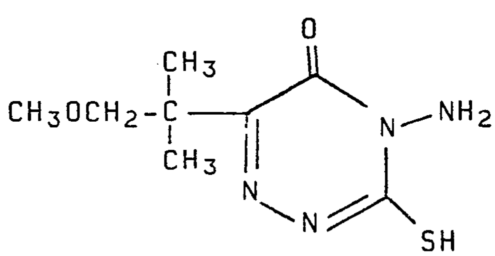

53g (0,5 Mol) Thiocarbohydrazid in 500 ml 1n Salzsäure werden auf 80-90°C erhitzt und tropfenweise mit einer Lösung von 89,1g rohem Dimethylmethoxymethyl-brenztraubensäure-N-tert.-butyl-amid (gemäß Stufe 1) in 150 ml Ethanol versetzt. Man läßt über Nacht bei 90 °C nachrühren, kühlt auf 10°C ab und saugt den kristallinen Niederschlag ab. Das feuchte Produkt wird über Natriumsulfat in Essigester umkristallisiert. Man erhält 37,3g (40 % der Theorie) 4-Amino-6-methoxy-tert.-butyl-3-mercapto-1,2,4-triazin-5-on vom Schmelzpunkt 167-68°C.

3. Stufe

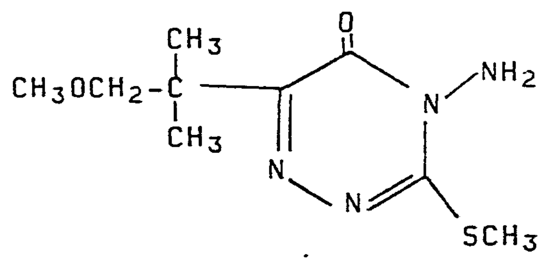

(1)

37,3g (0,16 Mol) 4-Amino-6-methoxy-tert.-butyl-3-mercapto-1,2,4-triazin-5-on (gemäß Stufe 2) werden in eine Lösung von 6,5g Natriumhydroxid in 150 ml Wasser eingetragen. Nach vollständiger Lösung des Produktes werden bei Raumtemperatur 28,4g (0,2 Mol) Methyljodid zugetropft. Nach beendeter Zugabe läßt man über Nacht bei Raumtemperatur nachrühren. Danach wird der entstehende Feststoff abgesaugt, mit Wasser gewaschen und getrocknet. Man erhält 19,3g (49 % der Theorie) 4-Amino-6-(methoxy-tert.-butyl)-3-methylthio-1,2,4-triazin-5-on (1) vom Schmelzpunkt 74-76°C.

Herstellung des Ausgangsproduktes

a)

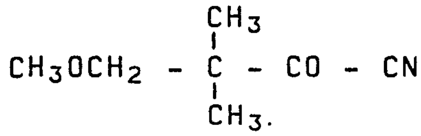

258g 93,8 %-iges 2,2-Dimethyl-3-methoxy-propionyl-chlorid werden auf 100-120°C erwärmt. Innerhalb 30 Minuten tropft man 163g 99 %-iges Trimethylsilylcyanid zu. Gleichzeitig wird das entstehende Trimethylsilylchlorid destillativ aus der Reaktionsmischung entfernt. Man rührt noch 30 Minuten bei 120°C nach und destilliert das Reaktionsprodukt. Man erhält 205g (88,2 % der Theorie) Methoxypivaloylcyanid vom Siedepunkt 61-64°C/16 mbar.

b)

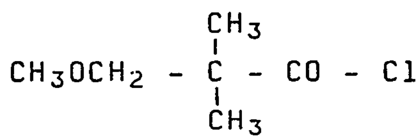

374,9 g (3,1 Mol) Thionylchlorid werden zu 279,4g (2,1 Mol) Dimethylmethoxymethyl-essigsäure getropft. Man läßt über Nacht bei Raumtemperatur rühren und erwärmt die Reaktionslösung dann auf 50°C, bis die Gasentwicklung beendet ist. Die Reaktionslösung wird fraktioniert. Man erhält 222,2g (70,3 % der Theorie) Methoxypivaloylchlorid vom Siedepunkt 46-47°C/26,6 mbar.

Beispiel 2

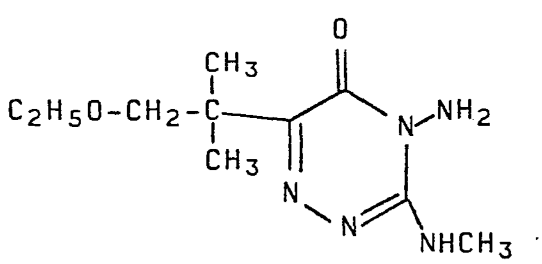

(2)

In eine Lösung von 1[illegible],og (0,2 Mol) Eisessig in 200 ml Isopropanol wird bei 5 bis 10°C 30 Minuten lang ein mittelstarker Gasstrom von Methylamin eingeleitet.

Diese Reaktionslösung wird anschließend mit 25,2g (0,098 Mol) 4-Amino-6-ethoxy-tert.-butyl-3-methylthio-1,2,4-triazin-5-on (Herstellung gemäß Beispiel 1) in 100 ml Isopropanol versetzt. Man läßt auf Raumtemperatur erwärmen und erhitzt anschließend 12 Stunden unter Rückfluß. Danach wird das Reaktionsgemisch eingeengt und der ölige Rückstand in Wasser eingerührt. Man extrahiert mit Methylenchlorid, trocknet über Natriumsulfat und engt ein. Der ölige Rückstand wird in Diisopropylether verrieben. Man erhält 5,8g (25 % der Theorie) 4-Amino-6-(ethoxy-tert.-butyl)-3-methylamino-1,2,4-triazin-5-on vom Schmelzpunkt 93-97°C.

Beispiel 3

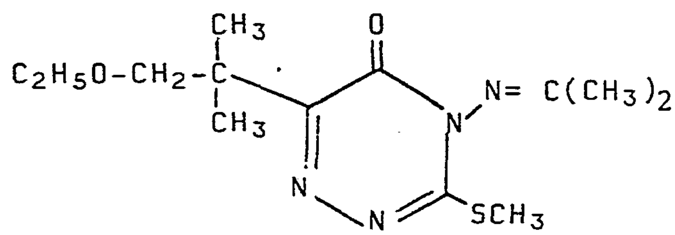

(3)

Zu 8,8 g (0,034 Mol) 4-Amino-6-ethoxy-tert.-butyl-3-methylthio-1,2,4-triazin-5-on (Herstellung gemäß Beispiel 1) und 150 ml Aceton werden bei Raumtemperatur 0,5 g p-Toluolsulfonsäure gegeben. Die Reaktionslösung wird 15 Stunden unter Rückfluß erhitzt. Danach läßt man abkühlen, engt ein, nimmt den Rückstand in Methylenchlorid auf, wäscht mit Wasser, trocknet über Natriumsulfat und engt ein. Nach Trennung über eine Kieselgelsäule (Kieselgel 60; Laufmittel: Ether/Petrolether 2:1) erhält man 1,6g (15,8 % der Theorie) 6-(Ethoxy-tert.-butyl)-4-isopropyl-ider amino-3-methylthio-1,2,4-triazin-5-on vom Brechungsindex $n_D^{20}$ = 1,551.

In analoger Weise und entsprechend dem erfindungsgemäßen Verfahren werden die in der nachfolgenden Tabelle 1 aufgeführten Verbindungen der Formel (I)

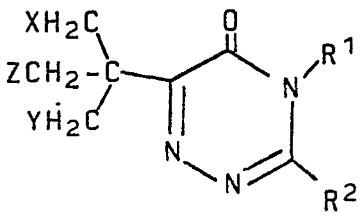

(I)

erhalten:

Tabelle 1

| Beispiel Nr. | X | Y | Z | $R^1$ | $R^2$ | Schmelzpunkt ($^oC$) oder Brechungsindex ($n_D^{20}$) |
|---|---|---|---|---|---|---|
| 4 | $C_2H_5O-$ | H | H | $-NH_2$ | $-SCH_3$ | $n_D^{20}$ = 1,534 |
| 5 | $C_2H_5O-$ | H | H | $-NH_2$ | $-N(CH_3)_2$ | $n_D^{20}$ = 1,533 |
| 6 | $CH_3O-$ | H | H | $-NH_2$ | $-N(CH_3)_2$ | 80-81 $^oC$ |
| 7 | $CH_3O-$ | H | H | $-NH_2$ | $-NHCH_3$ | 125-128 $^oC$ |
| 8 | $C_2H_5O-$ | H | H | $-NH_2$ | $-SC_2H_5$ | 80-81$^oC$ |
| 9 | $C_2H_5O-$ | H | H | $-NH_2$ | $-N(CH_3)(C_2H_5)$ | $n_D^{20}$ = 1,532 |
| 10 | $C_2H_5O-$ | H | H | $-NH_2$ | $-NH-CH(CH_3)(C_2H_5)$ | 100-103$^oC$ |
| 11 | $C_2H_5O-$ | H | H | $-N=C(CH_3)(CH_3)$ | $-N(CH_3)_2$ | $n_D^{20}$ = 1,5285 |

Tabelle 1 (Fortsetzung)

| Bei-spiel Nr. | X | Y | Z | $R^1$ | $R^2$ | Schmelzpunkt (°C) oder Brechungsindex ($n_D^{20}$) |
|---|---|---|---|---|---|---|
| 12 | $CH_3O-$ | H | H | 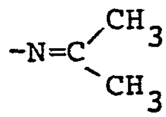 | $-N(CH_3)_2$ | $n_D^{20} = 1,529$ |
| 13 | $CH_3O-$ | H | H | 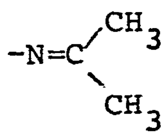 | $-SCH_3$ | 63 - 65 |

## Verwendungsbeispiele

In den nachfolgenden Beispielen wird die nachstehend angegebene Verbindung als Vergleichssubstanz eingesetzt:

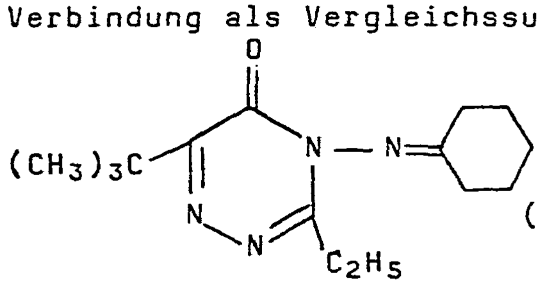

(A)

3-Ethyl-4-cyclohexylidenamino-6-tert.-butyl-1,2,4-triazin-5-on (bekannt aus DE-OS 22 38 206).

Beispiel A

Pre-emergence-Test

Lösungsmittel: 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in derZubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen z.B. die Verbindungen gemäß den Herstellungsbeispielen 1, 2, 3 und 4 neben einer besseren allgemeinen herbiziden Wirksamkeit insbesondere eine bessere Selektivität in Mais und Baumwolle als die aus dem Stand der Technik bekannte Verbindung (A).

Patentansprüche

1) Substituierte 6-Alkoxy-tert.-butyl-1,2,4-triazin-5-one der allgemeinen Formel I

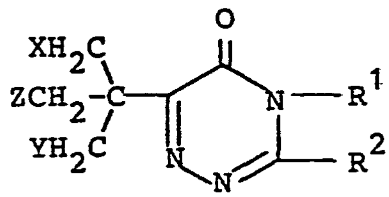

(I)

in welcher

$R^1$ für Amino oder die Gruppierung 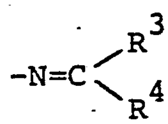 steht,

$R^2$ für Alkylthio, Alkylamino oder Dialkylamino steht,

$R^3$ für Wasserstoff oder Alkyl steht,

$R^4$ für Alkyl, Cycloalkyl, Cycloalkenyl oder gegebenenfalls substituiertes Phenyl steht,

X für Alkoxy steht sowie

Y und Z für Wasserstoff oder Alkoxy stehen.

2) Substituierte 6-Alkoxy-tert.-butyl-1,2,4-triazin-5-one der allgemeinen Formel (I) gemäß Anspruch 1, dadurch gekennzeichnet, daß

$R^1$ für Amino ($-NH_2$) oder die Gruppierung 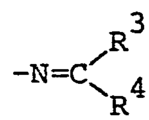 steht,

$R^2$ für Alkylthio mit 1 bis 4 Kohlenstoffatomen oder für Alkylamino oder Dialkylamino mit jeweils 1 bis 6 Kohlenstoffatomen in jedem Alkylteil steht,

$R^3$ für Wasserstoff oder Alkyl mit 1 bis 6 Kohlenstoffatomen steht,

$R^4$ für Alkyl mit 1 bis 6 Kohlenstoffatomen, Cycloalkyl mit 3 bis 6 Kohlenstoffatomen, Cycloalkenyl mit 5 oder 6 Kohlenstoffatomen oder für Phenyl, welches gegebenenfalls durch Halogen, Alkyl mit 1 bis 4 Kohlenstoffatomen, Halogenalkyl mit 1 bis 2 Kohlenstoff- und bis zu 5 gleichen oder verschiedenen Halogenatomen, Alkoxy oder Alkylthio mit jeweils 1 bis 2 Kohlenstoffatomen, Cyano oder Nitro substituiert sein kann, steht,

X für Alkoxy mit 1 bis 4 Kohlenstoffatomen steht und

Y und Z für Wasserstoff oder Alkoxy mit 1 bis 4 Kohlenstoffatomen stehen.

3) 4-Amino-6-(methoxy-tert.-butyl)-3-methylthio-1,2,4-triazin-5-on der Formel

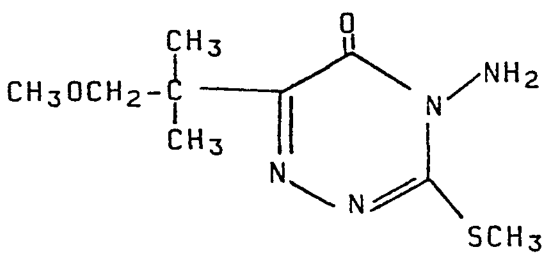

(1)

gemäß Anspruch 1.

4) 4-Amino-6-(ethoxy-tert.-butyl)-3-methylthio-1,2,4-triazin-5-on der Formel

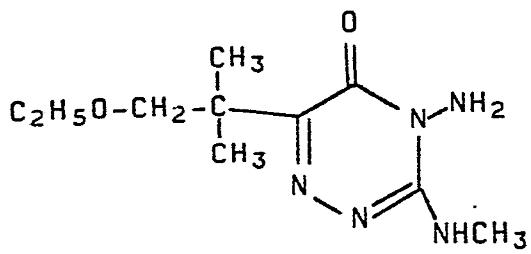

(2)

gemäß Anspruch 1.

5) 4-Amino-6-(ethoxy-tert.-butyl)-3-methylthio-1,2,4-triazin-5-on der Formel

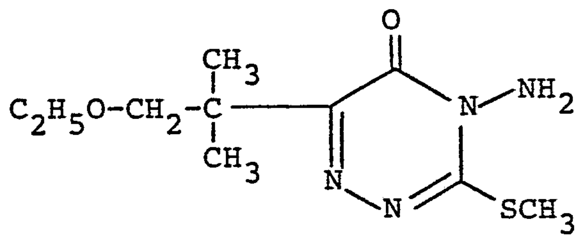

(4)

gemäß Anspruch 1.

6) 6-(Ethoxy-tert.-butyl)-4-isopropylidenamino-1,2,4-triazin-5-on der Formel

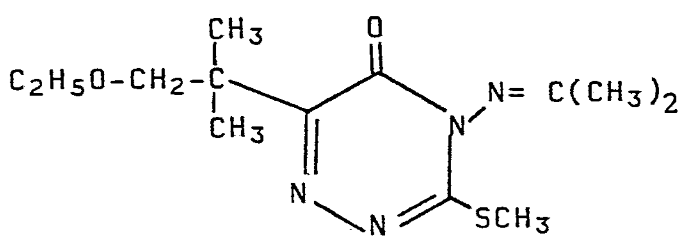

(3)

gemäß Anspruch 1.

7) Verfahren zur Herstellung von substituierten 6-Alkoxy-tert.-butyl-1,2,4-triazin-5-onen der allgemeinen Formel I

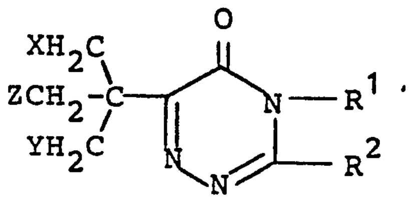

(I)

in welcher

$R^1$ für Amino oder die Gruppierung $-N=C\begin{smallmatrix}R^3\\R^4\end{smallmatrix}$ steht,

$R^2$ für Alkylthio, Alkylamino oder Dialkylamino steht

$R^3$ für Wasserstoff oder Alkyl steht,

$R^4$ für Alkyl, Cycloalkyl, Cycloalkenyl oder gegebenenfalls substituiertes Phenyl steht,

X für Alkoxy steht sowie

Y und Z für Wasserstoff oder Alkoxy stehen,

dadurch gekennzeichnet, daß man in einer <u>ersten Stufe</u> Alkoxypivaloylcyanide der Formel II

$$ZCH_2-\underset{CH_2Y}{\overset{CH_2X}{C}} - CO - CN \qquad (II)$$

in welcher

X,Y und Z die oben angegebene Bedeutung haben,

(a) gegebenenfalls in Gegenwart einer flüssigen Carbonsäure als Lösungsmittel, mit einer anorganischen Säure umsetzt oder

(b) an ein Carboniumion addiert, das aus einem Olefin, wie z.B. Isobutylen, oder einem tertiären Alkohol, wie z.B. tert.-Butanol, mit einer starken Säure, wie insbesondere Schwefelsäure, gebildet wird, und anschließend der Hydrolyse unterwirft

und

die hierbei entstehenden substituierten Trimethylbrenztraubensäureamide der Formel IIIa

$$\begin{array}{c} \quad\quad\quad CH_2X \\ \quad\quad\quad | \\ ZCH_2 - C - CO\text{-}CO - NHR \\ \quad\quad\quad | \\ \quad\quad\quad CH_2Y \end{array} \qquad (IIIa)$$

in welcher

X,Y und Z die oben angegebene Bedeutung haben und

R für Wasserstoff (erhältlich nach Variante a) oder Alkyl, insbesondere tert.-Butyl (erhältlich nach Variante b), steht,

in einer <u>zweiten Stufe</u> in an sich bekannter Weise entweder direkt in der anfallenden Lösung oder nach Zwischenisolierung, gegebenenfalls nach vorheriger Verseifung zu den freien substituierten Trimethylbrenztraubensäuren der Formel IIIb

$$\begin{array}{c} \quad\quad\quad CH_2X \\ \quad\quad\quad | \\ ZCH_2 - C - CO - COOH \\ \quad\quad\quad | \\ \quad\quad\quad CH_2Y \end{array} \qquad (IIIb)$$

in welcher

X,Y und Z die oben angegebene Bedeutung haben,

mit Thiocarbohydrazid der Formel IV

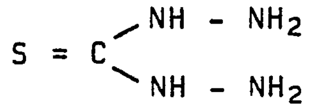

(IV)

in wässriger bzw. in wässrig-saurer Lösung, gegebenenfalls in Gegenwart eines organischen Verdünnungsmittels, zu 6-Alkoxy-tert.-butyl-3-mercapto-1,2,4-triazin-5-onen der Formel V

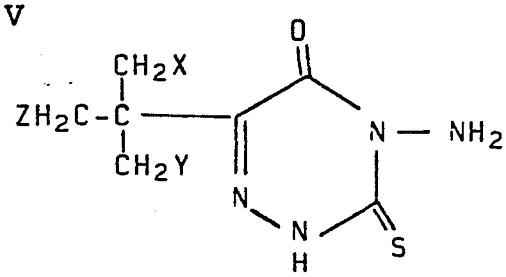

(V)

in welcher

X,Y und Z die oben angegebene Bedeutung haben,

umsetzt, diese in einer <u>dritten Stufe</u> in bekannter Weise in alkalischer Lösung mittels Alkylhalogenid, vorzugsweise Alkyljodid oder -bromid, zu 6-Alkoxy-tert.-butyl-3-alkylthio-1,2,4-triazin-5-onen der Formel Ia

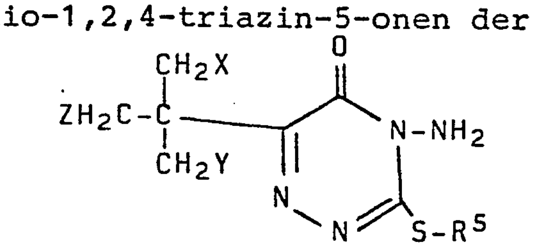

(Ia)

in welcher

X,Y und Z die oben angegebene Bedeutung haben und

$R^5$ für Alkyl, vorzugsweise mit 1 bis 4 Kohlenstoffatomen steht,

alkyliert, diese gegebenenfalls in einer <u>vierten Stufe</u> mit Aminen der Formel VI

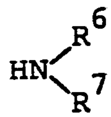

(VI)

in welcher

$R^6$ für Wasserstoff oder Alkyl, vorzugsweise mit 1 bis 6 Kohlenstoffatomen, steht und

$R^7$ für Alkyl, vorzugsweise mit 1 bis 6 Kohlenstoffatomen, steht,

in Gegenwart eines Verdünnungsmittels umsetzt und gegebenenfalls in einer <u>fünften Stufe</u> die gemäß der dritten bzw. vierten Stufe entstandenen 6-Alkoxy-tert.-butyl-4-amino-1,2,4-triazin-5-one der Formel Ib

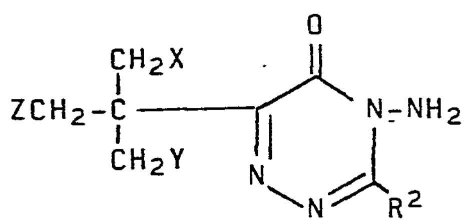

(Ib)

in welcher

$R^2$, X, Y und Z die oben angegebene Bedeutung haben,

mit Carbonylverbindungen der Formel VII

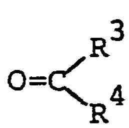

(VII)

i- welcher

$R^3$ und $R^4$ die oben angegebene Bedeutung haben,

gegebenenfalls in Gegenwart eines Verdünnungsmittels und gegebenenfalls in Gegenwart eines basischen oder sauren Katalysators umsetzt, wobei die Carbonylverbindungen der Formel (VII) gegebenenfalls in Form von Acetalen bzw. Ketalen eingesetzt werden können.

8) Herbizide Mittel, gekennzeichnet durch einen Gehalt an mindestens einem 6-Alkoxy-tert.-butyl-1,2,4-triazin-5-on der Formel (I) gemäß Anspruch 1 bzw. 7.

9) Verwendung von substituierten 6-Alkoxy-tert.-butyl-1,2,4-triazin-5-onen der Formel (I) gemäß Anspruch 1 bzw. 7 zur Bekämpfung von unerwünschtem Pflanzenwachstum.

10) Verfahren zur Herstellung von herbiziden Mitteln, dadurch gekennzeichnet, daß man substituierte 6-Alkoxy-tert.-butyl-1,2,4-triazin-5-one der Formel (I) gemäß Anspruch 1 bzw. 7 mit Streckmitteln und/oder oberflächenaktiven Mitteln vermischt.

Europäisches Patentamt

# EUROPÄISCHER RECHERCHENBERICHT

Nummer der Anmeldung 0074588

EP 82 10 7874

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | US-A-3 671 523 (K. WESTPHAL)<br>* Insgesamt; insbesondere Spalte 1, Zeile 45 * | 1,2,7-10 | C 07 D 253/06<br>A 01 N 43/64 //<br>C 07 C 121/34<br>C 07 C 103/153<br>C 07 C 59/125 |
| Y | DE-A-1 795 784 (BAYER)<br>* Insgesamt * | 1,2,7-10 | |
| Y | FR-A-2 308 628 (BAYER)<br>* Insgesamt * | 1,2,7-10 | |
| Y | US-A-4 058 526 (W. MERZ)<br>* Ansprüche * | 1,2,7 | RECHERCHIERTE SACHGEBIETE (Int. Cl. 3) |
| P,Y | EP-A-0 049 416 (BAYER)<br>* Insgesamt * | 1,2,7-10 | C 07 D 253/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 08-12-1982 | NUYTS A.M.K.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument